(19) Europäisches Patentamt
European Patent Office
Office européen des brevets

(11) Numéro de publication: **0 050 072**
**B1**

(12) # FASCICULE DE BREVET EUROPEEN

(45) Date de publication du fascicule du brevet: **27.12.84**

(21) Numéro de dépôt: **81401516.0**

(22) Date de dépôt: **30.09.81**

(51) Int. Cl.$^3$: **C 07 D 295/02,**
C 07 D 241/04,
C 07 D 295/08,
C 07 D 295/20,
C 07 D 295/18,
C 07 D 295/14,
C 07 D 295/06,
C 07 D 295/10,
A 61 K 31/495,
C 07 D 317/68, C 07 D 405/10

(54) **Nouvelles cyclopropylméthyl pipérazines, leur procédé de préparation et leur application en thérapeutique.**

(30) Priorité: **08.10.80 FR 8021527**

(43) Date de publication de la demande:
**21.04.82 Bulletin 82/16**

(45) Mention de la délivrance du brevet:
**27.12.84 Bulletin 84/52**

(84) Etats contractants désignés:
**AT BE CH DE GB IT LI LU NL SE**

(56) Documents cités:
**US-A-4 096 261**

**JOURNAL OF MEDICINAL CHEMISTRY, vol. 15, no. 6, 1972, WASHINGTON D.C. (US) W.J. IRWIN et al.: *"Alkyl derivatives of tetrahydroisoquinoline, 1-phenyl-piperazine, and 4-diphenylmethyl-piperidine"*, pages 690-692**

(73) Titulaire: **INNOTHERA**
**10, avenue Paul Vaillant Couturier**
**F-94110 Arcueil (FR)**

(72) Inventeur: **Robba, Max Fernand**
**4, rue Massillon**
**F-75004 Paris (FR)**
Inventeur: **Aurousseau, Michel**
**104, boulevard Arago**
**F-75013 Paris (FR)**

(74) Mandataire: **Kedinger, Jean-Paul et al**
**c/o Cabinet Malemont 42, avenue du Président Wilson**
**F-75116 Paris (FR)**

**EP 0 050 072 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

La présente invention concerne de nouvelles cyclopropylméthyl pipérazines, leur procédé de préparation et leur application en thérapeutique.

On connaît déjà de tels composés; il s'agit plus précisément du dichlorhydrate de la 1-cyclopropylméthyl-4-phényl-pipérazine [décrit dans Journal of Medicinal Chemistry, vol. 15, n° 6 (1972)] et de la 1-cyclopropylméthyl-pipérazine (décrite dans le brevet US—A—4 096 261). Le premier de ces composés est connu pour son activité neuroleptique; quant au second de ces composés, le brevet US—A—4 096 261 n'en donne aucune propriété pharmacologique.

Les nouvelles cyclopropylméthyl pipérazines selon l'invention répondent plus précisément à la formule générale:

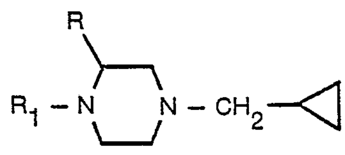 , $(A)_n$ [I]

dans laquelle:

—R représente un atome d'hydrogène ou un radical alkyle comportant de 1 à 4 atome de carbone;

—$R_1$ représente:

un atome d'hydrogène,

un radical alkyle linéaire ou ramifié comportant de 1 à 5 atomes de carbone,

un radical cycloalkylméthyle où le groupe cycloalkyle comporte de 3 à 6 atomes de carbone,

un groupement hydroxy-2 éthyle,

un groupement alkoxycarbonyle où le groupe alkoxy comporte 1 ou 2 atomes de carbone,

un groupement carboxy-2 propyle ou N-benzyl carbamoyl-2 propyle,

un noyau phényle éventuellement substitué par un groupe méthoxy, trifluorométhyle ou acétyle,

un groupe benzyle éventuellement substitué par un atome d'halogène et/ou par un groupe méthylènedioxy,

un groupe benzoyle éventuellement substitué par un groupe méthylènedioxy; par trois groupes méthoxy; par deux groupes méthoxy respectivement en positions 3 et 5 et un groupe acétoxy en position 4; ou par deux groupes méthoxy respectivement en positions 3 et 5 et un groupe éthoxycarbonyloxy en position 4,

un groupe cinnamoyle dont le noyau phényle est éventuellement substitué par un groupe trifluorométhyle, ou par un ou plusieurs groupe méthoxy,

un groupe phénoxyacétyle dont le noyau phényle est éventuellement substitué par un atome d'halogène ou un groupe méthoxy,

un noyau (benzothiényl-3) méthyle, ou

un radical de formule:

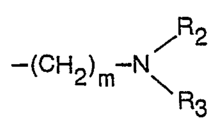

dans laquelle m = 2 ou 3 et $R_2$ et $R_3$ sont identiques et représentent alors un groupe alkyle ayant de 1 à 3 atomes de carbone, ou forment conjointement avec l'atome d'azote auquel ils sont liés, un radical choisi parmi les suivants: pyrrolidino, pipéridino, hexaméthylènimino, morpholino;

—A représente un composé physiologiquement acceptable choisi parmi les acide minéraux, les acides organiques, les halogénures d'alkyle et les halogénures d'aryle; et

— $n = 0$, 1, 2 ou 3;

$R_1$ ne pouvant toutefois représenter un noyau phényle ou un atome d'hydrogène quand $R = H$.

A titre de composés A préférés, on peut citer pour les acides minéraux, l'acide chlorhydrique, l'acide bromhydrique, l'acide sulfurique et l'acide phosphorique; pour les acides organiques, l'acide lactique, l'acide tartrique, l'acide pyruvique, l'acide glycolique, l'acide oxalique, l'acide citrique, l'acide malonique, l'acide malique, l'acide succinique, l'acide maléïque, l'acide fumarique, l'acide, embonique et l'acide méthane sulfonique; pour les halogénures d'alkyle, le bromure de méthyle, le bromure d'éthyle et le bromure de butyle; et pour les halogénures d'aryle, le bromure de benzyle.

Dans le cas où n dans la formule (I) prend la valeur zéro, les composés correspondants sont sous leur forme base et dans les cas où n prend la valeur 1, 2 ou 3, les composés correspondants sont sous la forme de sel d'ammonium.

Les composés de formule (I) dans laquelle $R_1$ représente un atome d'hydrogène, sont obtenus par condensation d'au plus 1 mole de bromométhylcyclopropane sur 1 mole de pipérazine de formule:

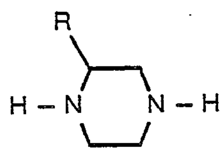

[II]

dans laquelle R désigne un radical alkyle comportant de 1 à 4 atomes de carbone, les composés résultants de formule:

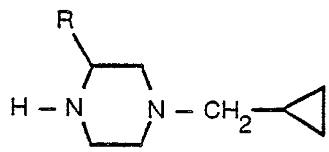

, $(A)_{n_1}$ [Ia]

où R a la même signification que dans la formule (II) et $n_1 = 0$, étant ensuite éventuellement salifiés par addition d'un composé de formule A définie dans la formule (I), ce qui conduit aux composés de formule:

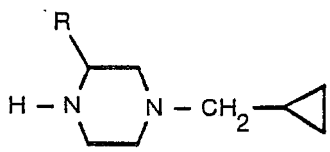

, $(A)_{n_2}$ [Ib]

où R et A ont la même signification que dans la formule (II) et la formule (I) respectivement et $n_2$ est égal à 1 ou 2 selon la quantité de composé A ajoutée.

La réaction de condensation ci-dessus est réalisée dans un solvant organique, inerte dans les conditions de la réaction et apte à solubiliser les réactifs, tel que par exemple l'acétate d'éthyle. Par ailleurs, on opèrera de préférence à une température pas trop élevée (par exemple à la température ambiante) pour éviter au maximum qu'une seconde molécule de bromométhylcyclopropane ne vienne se condenser sur le composé de formule (II), et en présence d'une base qui peut être soit minérale et du type carbonate dipotassique, soit organique et du type triéthylamine. Enfin, pour des questions de rendement, il est recommandé d'utiliser un excès de pipérazine de formule (II) par rapport au bromométhylcyclopropane, ces composés pouvant par exemple être dans un rapport molaire d'environ 1/0,35.

Les composés de formule (I) dans laquelle $R_1$ représente un groupe cyclopropylméthyle, sont quant à eux, obtenus par condensation d'au moins 2 moles de bromométhylcyclopropane respectivement sur 1 mole de pipérazine et 1 mole du composé de formule (II), les composés résultants de formule:

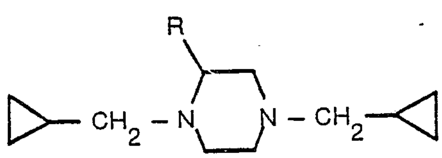

, $(A)_{n_1}$ [Ic]

où R a la même signification que dans la formule (I) et $n_1 = 0$, étant ensuite éventuellement salifiés par addition d'un composé de formule A déjà définie dans la formule (I), ce qui conduit aux composés de formule:

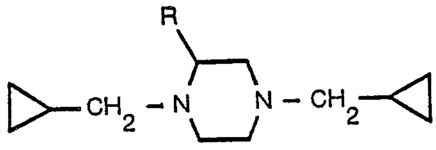 , $(A)_{n_2}$ [Id]

où R et A ont la même signification que dans la formule (I) et $n_2$ est étal à 1 ou 2 selon la quantité de composé A ajoutée.

La réaction de condensation peut être réalisée dans les mêmes conditions opératoires (solvant, base) que précédemment. Il est néanmoins préférable d'opérer à température plus élevée par exemple au reflux pour favoriser la double condensation du bromométhylcyclopropane; le solvant utilisé pourra alors être choisi parmi ceux ayant un point d'ébullition assez élevé, comme par exemple le N,N-diméthylformamide.

Les composés selon l'invention pour lesquels $R_1$ a la même signification que dans la formule (I), à l'exception des valeurs suivantes: H, phényle et phényle substitué par un groupe méthoxy, trifluorométhyle ou acétyle, sont pour leur part, obtenus en condensant la N-cyclopropylméthyl pipérazine ou une pipérazine de formule (Ia) avec un composé de formule:

$$R'_1\text{—}X \qquad \text{III}$$

dans laquelle X représente un atome d'halogène et $R'_1$ a la même signification que $R_1$ dans la formule (I), à l'exception des valeurs suivantes: H, phényle et phényle substitué par un groupe méthoxy, trifluorométhyle ou acétyle, ce qui conduit aux composés de formule:

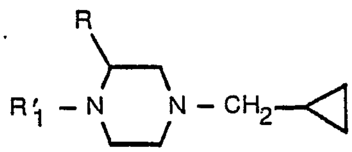 , $(A)_{n_1}$ [Ie]

où $R'_1$, et R ont la même signification que dans les formules (III) et (I) respectivement et $n_1 = 0$, ces composés de formule (Ie) étant ensuite éventuellement salifiés par addition d'un composé de formule A définie précédemment, pour conduire aux composés de formule:

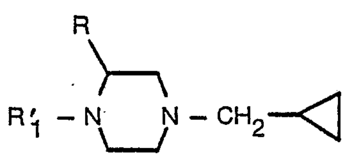 , $(A)_{n_3}$ [If]

où $R'_1$ et R ont la même signification que dans la formule (Ie), A a la même signification que dans la formule (I) et $n_3$ est égal à 1, 2 ou 3 selon la quantité de composé A ajoutée et/ou de la réactivité de la base de formule (Ie).

La condensation des composés de formules (Ia) et (III) est réalisée dans un solvant organique inerte dans les conditions de la réaction et apte à dissoudre les réactifs. Plus précisément, quand dans la formule (III), $R'_1$ contient un groupe carbonyle directement relié à X, il en résulte des composés $R'_1$—X généralement très réactifs et dans ce cas il est préférable de réaliser la condensation dans un solvant tel que l'acétate d'éthyle et à basse température, par exemple entre 0°C et la température ambiante. Quand ce même groupe $R'_1$ contient un groupe —$CH_2$— relié directement à X, les composés de formule (III) résultants sont moins réactifs et la condensation est alors effectuée dans un solvant tel que l'isopropanol et au reflux.

Enfin, les composés selon l'invention pour lesquels $R_1$ a la même signification que dans la formule (I), à l'exception des valeurs

H et 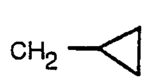

peuvent également être obtenus en condensant le bromométhylcyclopropane sur une pipérazine substituée de formule:

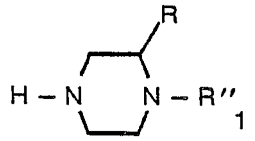

[IV]

dans laquelle R a la même signification que dans la formule (I) et $R''_1$ a la même signification que $R_1$ dans la formule (I), à l'exception des valeurs

$-H$ et $-CH_2-$ 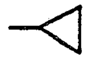

les composés résultants de formule:

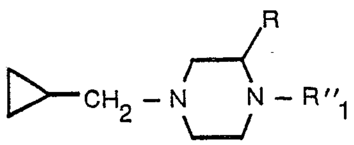 , $(A)_{n_1}$

[Ig]

où R et $R''_1$, ont la même signification que dans la formule (IV) et $n_1 = 0$, étant éventuellement salifiés par addition d'un composé de formule (A) déjà définie précédemment, ce que conduit aux composés de formule:

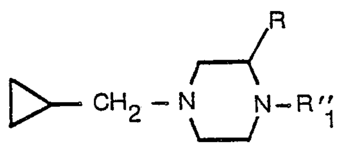 , $(A)_{n_3}$

[Ih]

dans laquelle R et A ont la même signification que dans la formule (I), $R''_1$ a la même signification que dans la formule (IV) et $n_3$ est égal à 1, 2 ou 3 selon la quantité de composé A ajoutée.

La réaction de condensation est réalisée dans un solvant organique, inerte dans les conditions de la réaction et apte à dissoudre les réactifs, tel que le N,N-diméthylformamide.

Par ailleurs, on opèrera de préférence à reflux et en présence d'une base minérale ou organique, du type carbonate dipotassique ou triéthylamine.

La salification facultative des bases de formules (Ia), (Ic), (Ie) et (Ig) est réalisée selon les techniques classiques, par exemple par action du composé de formule (A) en solution dans un solvant approprié, sur la base éventuellement en solution dans un solvant organique approprié.

Les préparations ci-après sont données à titre d'exemple pour illustrer l'invention.

Exemple 1:
Dicyclopropylméthyl-1,4 pipérazine (Ic)

*Numéro de code:* 3

Un mélange de 51,6 g de pipérazine anhydre, de 216 g de bromométhylcyclopropane et de 128 g de carbonate dipotassique dans 400 ml de diméthylformamide, est chauffé pendant 30 heures à 110°C avec agitation. Après refroidissement, on filtre, élimine le solvant et les réactifs non transformés, sous vide, puis rectifie. On recueille le composé recherché sous la forme d'une huile incolore.

. Rendement : 60%
. $E_{18} = 137—140°C$

Par le même procédé, mais à partir des réactifs correspondants, on obtient la base correspondant au composé de numéro de code 13 répertoriée dans le tableau I ci-après.

*Le bis-chlorhydrate* (Id) [numéro de code: 3a] de la base (Ic) ainsi préparée est obtenu en ajoutant une solution de 120 ml d'éthanol absolu saturé d'acide chlorhydrique gazeux à une solution de 30 g de la base obtenue ci-dessus dans 700 ml d'éthanol. Il en résulte le bis-chlorhydrate attendu, recristallisable dans l'éthanol. Ce sel se présente sous la forme de cristaux blancs et possède un point de fusion de 286°C.

Par la même procédé, mais à partir de la base correspondante, on obtient le numéro de code 13 répertorié dans le tableau I ci-après.

*Le bis-oxalate* (Id) [numéro de code: 3b] de la base (Ic) préparée ci-dessus est obtenu en chauffant au reflux pendant 15 minutes une solution de 5 g de dicyclopropylméthyl-1,4 pipérazine et de 5 g d'acide oxalique dans 150 ml d'acétone. On isole ainsi le sel attendu, recristallisable dans l'eau. Il se présente sous la forme de cristaux blancs et fond à 266°C.

*Le bis-citrate* (Id) [numéro de code: 3c] de la dicyclopropylméthyl-1,4 pipérazine base est obtenu en chauffant au reflux pendant 15 minutes une solution de 5 g de cette base et de 5 g d'acide citrique dans l'acétone. Il en résulte le sel attendu, recristallisable dans l'eau. Il se présente sous la forme de cristaux blancs fondant à 163°C.

*Le bis-bromométhylate* (Ic) [numéro de code: 3d] de la dicyclopropylméthyl-1,4 pipérazine base est obtenu en faisant passer pendant 15 minutes un courant de bromure de méthyle dans une solution de 5 g de ladite base dans 100 ml d'acétone. On concentre, filtre et recristallise le produit obtenu dans l'acétonitrile. On isole ainsi le sel attendu qui se présente sous la forme de cristaux blancs fondant à 225°C.

Exemple 2:
Cyclopropylméthyl-1 méthyl-3 pipérazine (Ia)

*Numéro de code:* 23

Un mélange de 46 g de bromométhylcyclopropane, 100,0 g de méthyl-3 pipérazine anhydre et 23,4 g de carbonate dipotassique dans 400 ml d'acétate d'éthyle est agité pendant 5 heures à la température ambiante. On essore, lave la phase organique avec une solution aqueuse de soude 4N, élimine le solvant et distille sous vide. Il en résulte le produit attendu.

Exemple 3:
Cyclopropylméthyl-1 carbéthoxy-4 pipérazine

*Numéro de code:* 6

On chauffe au reflux pendant 6 heures, sous agitation, un mélange de 25,5 g de bromométhylcyclopropane, 30,5 g de N-carbéthoxypipérazine (IV) et 2,5 g de carbonate dipotassique dans 25 ml de N,N-diméthylformamide. On filtre, élimine le solvant et distille sous vide. On isole ainsi le produit désiré qui se présente sous le forme d'une huile incolore dont le point d'ébullition sous 11 mm de Hg est de 142—148°C.

Par le même procédé, mais à partir des réactifs correspondants, on obtient les bases correspondant aux composés numéros de code 2, 4, 5, 7, 8, 10 à 12, 14, 35, à 37, 40 et 41 répertoriés dans le tableau I ci-après.

Exemple 4:
Chlorhydrate de cyclopropylméthyl-1 p-fluorophénoxyacétyl-4 pipérazine

*Numéro de code:* 22

Une solution de 9,64 g de chlorure de para-fluorophénoxyacétyle (III) dans 25 ml d'acétate d'éthyle est versée sous agitation à 0°C dans une solution de 7,5 g de cyclopropylméthylpipérazine dans 25 ml d'acétate d'éthyle. On agite 1 heure à 20°C après la fin de l'addition. On essore, lave à l'éther et sèche le dérivé désiré qui est obtenu sous forme de chlorhydrate. On le recristallise dans un mélange éthanol absolu/éther éthylique (1/1), ce qui conduit à l'obtention de cristaux blancs fondant à 139°C.

Par le même procédé, mais à partir des réactifs correspondants, on obtient les composés de numéros de code 15 à 21, 38 et 39 répertoriés dans le tableau I ci-après.

Exemple 5:
Trichlorhydrate de cyclopropylméthyl-1 méthyl-3 (N-pipéridino-2 éthyl)-4 pipérazine

*Numéro de code:* 27

Une solution de 4,62 g de cyclopropylméthyl-1 méthyl-3 pipérazine et de 4,90 g de N-pipéridino-2 chloroéthane dans 100 ml d'isopropanol est chauffée au reflux pendant 17 heures. Puis, en évapore à sec sous vide, reprend le résidu dans la soude 2N et extrait à l'éther éthylique. On élimine le solvant et transforme le produit résultant en chlorhydrate avec une solution d'éthanol saturé d'acide chlorhydrique gazeux anhydre. Le trichlorhydrate obtenu et recristallisé dans l'éthanol à 95° fond à 264°C.

Par le même procédé, mais à partir des réactifs correspondants, on obtient les composés de numéros de code 24 à 26 et 28 à 34 répertoriés dans le tableau I ci-après.

TABLEAU I

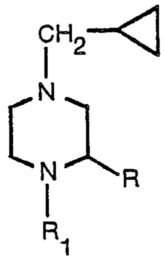

, $(A)_n$ (I)

| Numéro de Code | R | $R_1$ | $(A)_n$ | Solvant de cristallisation | Point de fusion (°C) | Rendement (%) |
|---|---|---|---|---|---|---|
| | | | | | | |
| | | | | | | |
| 2 | ,, | $CH_3$ | 2HCl | Acétonitrile/éthanol 95° (1/9) | 238 | 30 |
| 3 | ,, | $-CH_2$ 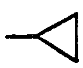 | Base | – | 137–140* | 60 |
| 3a | ,, | $-CH_2$ 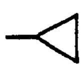 | 2HCl | Ethanol | 286 | 65 |
| 3b | ,, | ,, | $2(COOH)_2$ | Eau | 266 | 40 |
| 3c | ,, | ,, | Bis-citrate | ,, | 163 | 30 |
| 3d | ,, | ,, | Bis-bromo-méthylate | Acétonitrile | 225 | 75 |
| 3e | H | $-CH_2$ 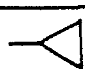 | bis-sulfo-méthylate | Ethanol absolu | 252 | 30 |
| 3f | ,, | ,, | bis-lactate | Acétonitrile | 78 | 30 |
| 3g | ,, | ,, | bis-tartrate | Ethanol absolu | 93 | 50 |
| 3h | ,, | ,, | bis-pyruvate | Ethanol à 95° | 191 | 25 |

*Point d'ébullition à 18 mm/Hg.

TABLEAU I (Suite)

| Numéro de Code | R | $R_1$ | $(A)_n$ | Solvant de cristal-lisation | Point de fusion (°C) | Rende-ment (%) |
|---|---|---|---|---|---|---|
| 3i | H | $-CH_2$ 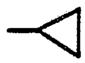 | bis-maléate | Ethanol à 95° | 224 | 50 |
| 3j | ,, | ,, | bis-glycolate | Ethanol absolu | 120 | 80 |
| 4 | ,, | $-(CH_2)_4CH_3$ | 2HCl | ,, | >300 | 50 |
| 5 | ,, | $-CH_2CH_2OH$ | 2HCl | Ethanol absolu/ Acéto-nitrile (1/1) | 191 | 20 |
| 6 | ,, | $-COOC_2H_5$ | Base | – | 142–148* | – |
| 6a | ,, | $-COOC_2H_5$ | HCl | Acéto-nitrile | 192 | 70 |
| 7 | ,, | 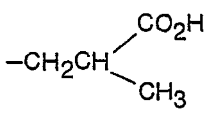 | 2HCl | Ethanol absolu | 232 | 35 |
| 8 | ,, | 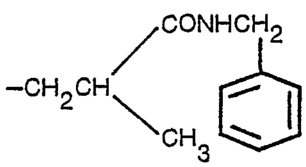 | $2(COOH)_2$ | Ethanol à 95° | 169,9 | 30 |
| | | | | | | |
| 10 | ,, | 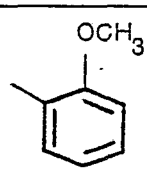 | 2HCl | Acéto-nitrile | 202 | 50 |
| 11 | ,, | 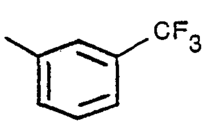 | HCl | Acéto-nitrile | 176 | 48 |

*Point d'ébullition à 11 mg/Hg.

TABLEAU I (Suite)

| Numéro de Code | R | $R_1$ | $(A)_n$ | Solvant de cristal-lisation | Point de fusion (°C) | Rende-ment (%) |
|---|---|---|---|---|---|---|
| 12 | H | 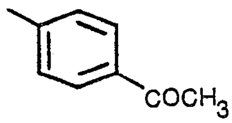 | HCl | Ethanol absolu | 183 | 20 |
| 12a | ,, | 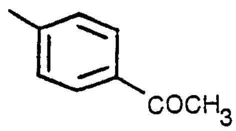 | HBr | Ethanol absolu | 232 | 30 |
| 13 | $CH_3$ | 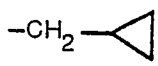 | 2HCl | Ethanol à 95° | 246,6 | 70 |
| 14 | H | 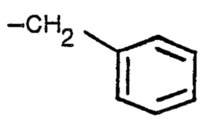 | 2HCl | Ethanol à 95° | 250 | 40 |
| 15 | ,, | 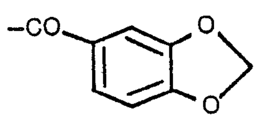 | HCl | Acéto-nitrile | 231 | 45 |
| 16 | ,, | 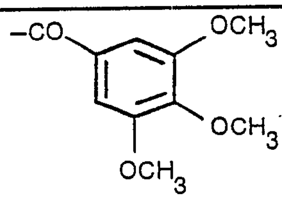 | $(COOH)_2$ | Dioxanne/ Eau (3/1) | 260 | 45 |
| 17 | ,, | 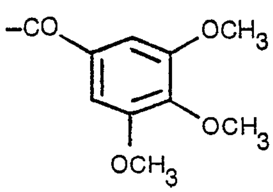 | HCl | Ethanol absolu | 201 | 35 |
| 18 | ,, | 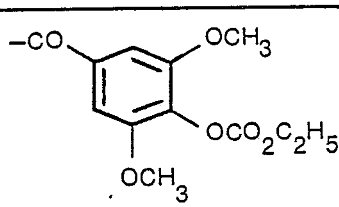 | HCl | Ethanol absolu | 230 | 35 |

TABLEAU I (Suite)

| Numéro de Code | R | $R_1$ | $(A)_n$ | Solvant de cristallisation | Point de fusion (°C) | Rendement (%) |
|---|---|---|---|---|---|---|
| 19 | H | 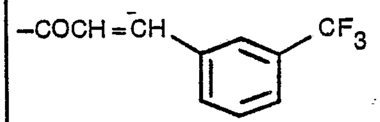 | HCl | Acéto-nitrile | 215 | 40 |
| 20 | ,, | 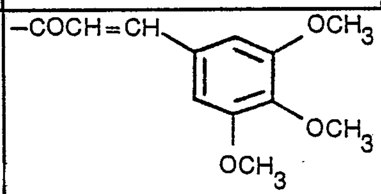 | HCl | Ethanol à 95° | 221 | 45 |
| 21 | ,, | 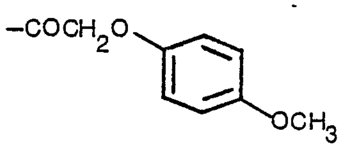 | HCl | Ethanol absolu/Ether éthylique (1/1) | 168 | 50 |
| 22 | ,, | 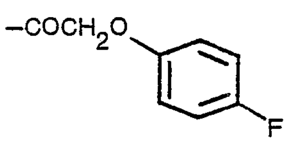 | HCl | ,, | 139 | 30 |
| 23 | $CH_3$ | H | 2HCl | Ethanol à 95° | 238 | 60 |
| 24 | ,, | $-CH_2CH_2N(CH_3)_2$ | $2(COOH)_2$ | Ethanol à 95° | 180 | 35 |
| 25 | ,, | $-CH_2CH_2N(C_2H_5)_2$ | $(COOH)_2$ | Ethanol a 95° | 201 | 60 |
| 26 | ,, | 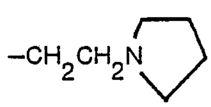 | $2(COOH)_2$ | Ethanol à 95° | 195 | 45 |
| 27 | ,, | 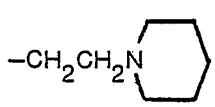 | 3HCl | Ethanol à 95° | 264 | 60 |

TABLEAU I (Suite)

| Numéro de Code | R | $R_1$ | $(A)_n$ | Solvant de cristallisation | Point de fusion (°C) | Rendement (%) |
|---|---|---|---|---|---|---|
| 28 | $CH_3$ | 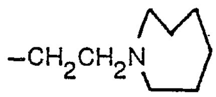 | $2(COOH)_2$ | Ethanol | 195 | 55 |
| 29 | ,, | 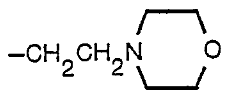 | $2(COOH)_2$ | Ethanol à 80° | 211 | 65 |
| 30 | ,, | $-CH_2CH_2CH_2N(C_2H_5)_2$ | $3(COOH)_2$ | Ethanol à 95° | 148 | 45 |
| 31 | ,, | 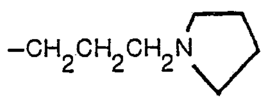 | $3(COOH)_2$ | Ethanol à 95° | 262 | 60 |
| 32 | ,, | 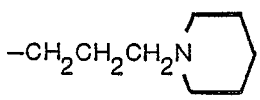 | $3(COOH)_2$ | Ethanol à 95° | 144 | 60 |
| 33 | ,, | 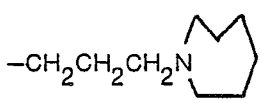 | $3(COOH)_2$ | Ethanol à 95° | 149 | 65 |
| 34 | ,, | 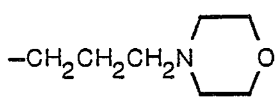 | $3(COOH)_2$ | Ethanol à 95° | 191 | 60 |
| 35 | ,, | 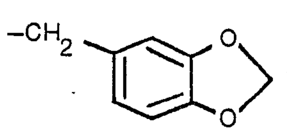 | $2(COOH)_2$ | Ethanol à 90° | 160 | 60 |
| 36 | ,, | 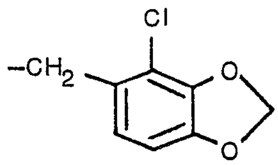 | $2(COOH)_2$ | Ethanol à 95° | 114 | 60 |

TABLEAU I (Suite)

| Numéro de Code | R | $R_1$ | $(A)_n$ | Solvant de cristallisation | Point de fusion (°C) | Rendement (%) |
|---|---|---|---|---|---|---|
| 37 | $CH_3$ | 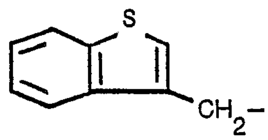 | $2(COOH)_2$ | Ethanol à 95° | 169 | 58 |
| 38 | ,, | 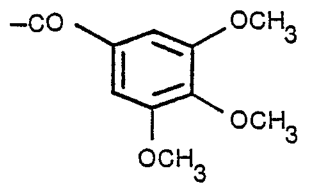 | $2(COOH)_2$ | Ethanol à 95° | 195 | 55 |
| 39 | ,, | 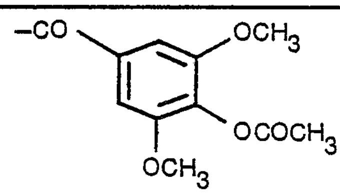 | $(COOH)_2$ | Ethanol à 95° | 158 | 45 |
| 40 | H | 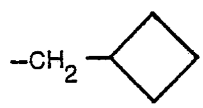 | HCl | Ethanol absolu | >300 | 40 |
| 41 | H | 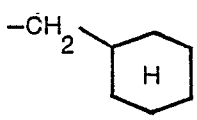 | HCl | Ethanol absolu | >300 | 50 |

L'étude des composés selon l'invention a permis de mettre en évidence une activité cardiotrope, notamment inotrope positive.

L'activité inotrope a été révélée par l'étude de l'action desdits composés sur la force contractile de l'oreillette gauche isolée du cobaye. La technique utilisée est celle de P. Lumley, K.J. Broadley et G.P. Lévy décrite dans Cardiovascular Research, 11, 17—25, 1977. Elle permet l'étude de la fonction inotrope $\beta_1$ cardiaque; les récepteurs $\beta_1$ étant cardiaques chez le cobaye comme chez l'homme, les résultants obtenus chez le cobaye sont avantageusement transposables chez l'homme.

Des cobayes, de sexe indifférent, son assommés et saignés; l'oreillette gauche est rapidement prélevée et aussitôt immergée dans une solution de Kreibs-Henseleit, thermostatée à la température de 36°C et aérée par un mélange de $O_2$ et $CO_2$ (95% et 5%). Deux fils de lin sont attachés à ses extrémités: l'un a pour fonction de mettre en contact l'oreillette avec une électrode d'argent négative de stimulation, l'autre sert à relier la préparation à un myographe isométrique (F—50 NARCO) puis à un système d'enregistrement (Physiograph M—K III NARCO).

Un délai de 60 minutes est nécessaire pour assurer la stabilité de la préparation.

Pendant toute la durée de l'essai, l'oreillette est entraînée électriquement par un neurostimulateur (Equipement Industriel II): voltage: supramaximum — largeur:5 milli/sec. — fréquence:90 à 120 batt./min.

Les agonistes, produits de référence ou composés selon l'invention, sont ajoutés au milieu nutritif suivant la méthode cumulative de VAN ROSSUM (Arch. Inter. Pharmacodyn., 143, 299—330, 1963).

Après chaque quantité donnée d'agoniste ajoutée, on attend 3 minutes avant de rajouter une nouvelle quantité donnée du produit à étudier suivant la méthode décrite ci-dessus. La réponse de l'organe à chaque concentration a été appréciée par l'augmentation consécutive de la force contractile étalonnée en gramme. Celle-ci a été exprimée en pourcentage de l'augmentation maximale (100%) pour chaque composé et une courbe dose-action a été tracée. La quantification de l'effet agoniste a été effectuée par le calcul du $pD_2$ (E.J. Ariens and J.M. Van Rossum., Arch. Intern. Parmacodyn. CX, n° 2, 2, 19 et Arch. Inter. Pharmacodyn. 143, 299—330, 1963), qui caractérise l'affinité du composé testé pour les récepteurs.

L'effet agoniste est également caractérisé par l'activité intrinsèque ($\alpha$) qui définit la puissance relative de l'agoniste par rapport à un standard qui a été, dans les essais, la dopamine dont l'activité intrinsèque est arbitrairement $\alpha = 1$.

Pour chaque composé étudié, ont été déterminées au minimum trois courbes dose-action, donc trois $pD_2$, en alternant avec le standard (dopamine). Les résultats obtenus sont répertoriés dans le tableau II ci-après, étant entendu que:

—plus $pD_2$ est élevé et plus l'affinité du composé testé pour les récepteurs est marquée, et que

—plus $\alpha$ est élevé, meilleure est l'activité intrinsèque.

On a par ailleurs procédé à l'étude toxicologique des composés selon l'invention.

Plus précisément, on a étudié la toxicité aiguë par voie veineuse chez la souris (10 animaux par lot), de sexe mâle, E.O.P.S. de souche SWISS (élevage EVIC-CEBA).

Les composés à tester ont été solubilisés dans du sérum physiologique à des concentrations variables et les solutions obtenues ont été injectées par voie veineuse à raison de 10 ml/kg de poids corporel. L'administration a été faite en 20 secondes.

Les animaux sont gardés en observation clinique pendant une durée de trois heures après l'administration, puis quotidiennement pendant 7 jours.

Les doses léthales 50 ($DL_{50}$) sont calculées par la méthode de LITCHFIELD et WILCOXON (J. Pharmacol. Exp. Therap., 96—99, 1949), à partir des diverses mortalités constatées.

Les résultats de cette étude sont également répertoriés dans le tableau II ci-après.

TABLEAU II

| Numéro de code du composé testé | Action inotrope | | *Toxicité — DL50 (mg/kg/i.v.) |
|---|---|---|---|
| | pD2 | $\alpha$ | |
| 2 | 3,95 | 0,48 | 320 (313–327) |
| 3a | 4,52 | 0,69 | 110 (105–116) |
| 4 | – | – | 37,5 (33,0–42,0) |
| 5 | – | – | 460 (439–482) |
| 6a | 2,90 | 0,58 | 41,0 (35,9–46,8) |
| 7 | – | – | 190 (185–195) |
| 8 | 4,27 | 0,57 | 245 (240–250) |
| 10 | – | – | 34,0 (32,0–37,0) |
| 11 | – | – | 30,0 (24,2–36,3) |
| 12 | 4,26 | 0,82 | 90,0 (86,0–95,0) |
| 13 | 5,44 | 0,36 | 104 (99,7–108,4) |
| 14 | – | – | 46,0 (41,4–51,2) |
| 15 | 3,44 | 0,88 | 155 (149–161) |

*Limite de confiance à 95%.

TABLEAU II (Suite)

| Numéro de code du composé testé | Action inotrope | | *Toxicité – DL50 (mg/kg/i.v.) |
|---|---|---|---|
| | pD2 | α | |
| 16 | 5,06 | 0,52 | 100 mg/kg = 30% de mortalité |
| 17 | 3,50 | 0,19 | 245 (236–255) |
| 18 | – | – | 225 (219–232) |
| 19 | – | – | 95,0 (90,0–100,0) |
| 20 | 3,11 | 0,49 | 52,0 (47,2–57,2) |
| 21 | 4,65 | 0,69 | 115 (108–122) |
| 22 | 4,75 | 0,48 | 100 (95–106) |
| 23 | 3,75 | 0,75 | 280 (274–286) |
| 24 | – | – | 200 (193–208) |
| 25 | – | – | 150 (143–158) |
| 26 | 4,85 | 0,32 | 158 (153–163) |
| 27 | 4,32 | 0,40 | 155 (149–161) |
| 28 | – | – | 95 (89–101) |
| 29 | – | – | 250 (236–265) |
| 30 | – | – | 135 (129–141) |
| 31 | 4,57 | 0,25 | 165 (160–170) |
| 32 | 6,28 | 0,12 | 162 (152–173) |
| 33 | – | – | 130 (123–137) |
| 34 | 5,29 | 0,24 | 215 (209–221) |
| 35 | 5,28 | 0,17 | 77,0 (71,3–83,1) |
| 36 | – | – | 65,0 (61,0–70,0) |
| 37 | – | – | 40,0 (34,0–47,0) |
| 38 | – | – | 130 (122–139) |
| 39 | 4,59 | 0,69 | 225 (219–231) |

*Limite de confiance à 95%

L'ensemble des études présentées ci-dessus montre l'intérêt des composés de formule (I) selon l'invention en thérapeutique humaine et animale, notamment comme agents pour le traitement des maladies cardiaques et en particulier des défaillances cardiaques et des troubles se caractérisant par un bas débit cardiaque.

Les composés selon l'invention peuvent être présentés en vue de l'administration orale ou parentérale chez l'homme et les animaux, éventuellement en association avec les excipients appropriés à ces voies.

Ainsi, par exemple, ils peuvent être présentés sous la forme de comprimés, de capsules, de gélules ou de solutions injectables.

La présente invention comprend donc l'application en thérapeutique des composés de formule (I), ainsi que les compositions pharmaceutiques contenant un ou plusieurs de ces composés en combinaison avec un excipient approprié.

La posologie quotidienne peut, selon les cas, aller de 100 mg à 1 g.

Un exemple de composition pharmaceutique administrable par voie orale est le suivant:

| | |
|---|---|
| Dicyclopropylméthyl-1,4 pipérazine, bis-chlorhydrate | 250 g |
| Lactose | 50 g |
| Amidon | 45 g |
| Stéarate de magnésium | 5 g |

pour un comprimé pesant 350 mg

**Revendications**

1. Cyclopropylméthyl pipérazines, caractérisées en ce qu'elles répondent à la formule générale:

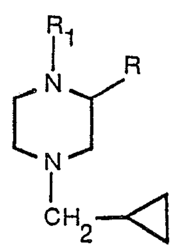 , $(A)_n$ [I]

dans laquelle:

— R représente un atome d'hydrogène ou un radical alkyle comportant de 1 à 4 atomes de carbone;

— $R_1$ représente:

un atome d'hydrogène,

un radical alkyle linéaire ou ramifié comportant de 1 à 5 atomes de carbone,

un radical cycloalkylméthyle où le groupe cycloalkyle comporte de 3 à 6 atomes de carbone,

un groupement hydroxy-2 éthyle,

un groupement alkoxycarbonyle où le groupe alkoxy comporte 1 ou 2 atomes de carbone,

un groupement carboxy-2 propyle ou N-benzyl carbamoyl-2 propyle,

un noyau phényle éventuellement substitué par un groupe méthoxy, trifluorométhyle ou acétyle,

un groupe benzyle éventuellement substitué par un atome d'halogène et/ou par un groupe méthylènedioxy,

un groupe benzoyle éventuellement substitué par un groupe méthylènedioxy; par trois groupes méthoxy; par deux groupes méthoxy respectivement en positions 3 et 5 et un groupe acétoxy en position 4; ou par deux groupes méthoxy respectivement en positions 3 et 5 et un groupe éthoxycarbonyloxy en position 4,

un groupe cinnamoyle dont le noyau phényle est éventuellement substitué par un groupe trifluorométhyle, ou par un ou plusieurs groupe méthoxy,

un groupe phénoxyacétyle dont le noyau phényle est éventuellement substitué par un atome d'halogène ou un groupe méthoxy,

un noyau (benzothiényl-3) méthyle, ou

un radical de formule:

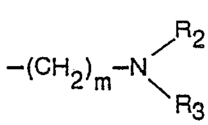

dans laquelle m = 2 ou 3 et $R_2$ et $R_3$ sont identiques et représentent alors un groupe alkyle de 1 à 3 atomes de carbone, ou forment conjointement avec l'atome d'azote aquel ils sont liés, un radical choisi parmi les suivants: pyrrolidino, pipéridino, hexaméthylènimino, morpholino;

—A représente un composé physiologiquement acceptable choisi parmi les acides minéraux, les acides organiques, les halogénures d'alkyle et les halogénures d'aryle; et

—n = 0, 1, 2 ou 3;

$R_1$ ne pouvant toutefois représenter un atome d'hydrogène ou un noyau phényle quand R = H.

2. Cyclopropylméthyl pipérazines selon la revendication 1, caractérisées en ce que A est choisi dans le groupe comprenant l'acide chlorhydrique, l'acide bromhydrique, l'acide sulfurique, l'acide phosphorique, l'acide lactique, l'acide tartrique, l'acide pyruvique, l'acide glycolique, l'acide oxalique, l'acide citrique, l'acide malonique, l'acide malique, l'acide succinique, l'acide maléïque, l'acide fumarique, l'acide embonique, l'acide méthane sulfonique, le bromure de méthyle, le bromure d'éthyle, le bromure de butyle, le bromure de benzyle.

3. Medicament notamment pour le traitement des défaillances cardiaques et des troubles dus à un bas débit cardiaque, caractérisé en ce qu'il est constitué par au moins un composé selon la revendication 1 ou 2.

4. Composition médicamenteuse, caractérisée en ce qu'elle contient au moins un composé selon la revendication 1 ou 2, en association avec un support pharmaceutiquement acceptable.

5. Procédé de préparation des composés de formule (I) dans laquelle $R_1$ représente un atome d'hydrogène, caractérisé en ce qu'il consiste dans la condensation d'au plus 1 mole de bromométhylcyclopropane sur 1 mole de pipérazine de formule:

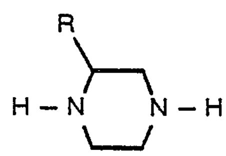

[II]

dans laquelle R désigne un radical alkyle comportant de 1 à 4 atomes de carbone, les composés résultants de formule:

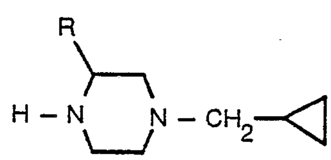 , $(A)_{n_1}$

[Ia]

où R a la même signification que dans la formule (II) et $n_1 = 0$, étant ensuite éventuellement salifiés par addition d'un composé de formule A définie dans la revendication 1, ce qui conduit aux composés de formule:

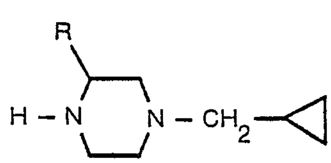 , $(A)_{n_2}$

[Ib]

où Ra la même signification que dans la formule (II), A a la même signification que dans la revendication 1 et $n_2$ est égal à 1 ou 2 selon la quantité de composé A ajoutée.

6. Procédé selon la revendication 5, caractérisé en ce que la réaction de condensation est réalisée à température ambiante, dans un solvant organique, inerte dans les conditions de la réaction et apte à solubiliser les réactifs et en présence d'une base.

7. Procédé selon la revendication 6, caractérisé en ce que le solvant organique est l'acétate d'éthyle et la base est le carbonate dipotassique ou la triéthylamine.

8. Procédé de préparation des composés de formule (I) dans laquelle $R_1$ représente un groupe cyclopropylméthyle, caractérisé en ce qu'il consiste dans la condensation d'au moins 2 moles de bromométhylcyclopropane respectivement sur 1 mole de pipérazine et 1 mole de composé de formule (II), les composés résultants de formule:

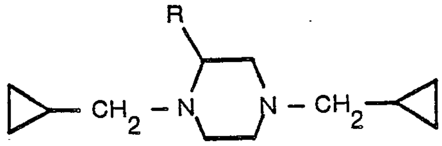 , $(A)_{n_1}$ [Ic]

où R a la même signification que dans la revendication 1 et $n_1 = 0$, étant ensuite éventuellement salifiés par addition d'un composé de formule A définie dans la revendication 1, ce qui conduit aux composés de formule:

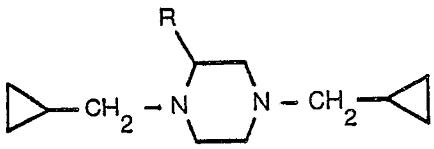 , $(A)_{n_2}$ [Id]

où R et A ont la même signification que dans la revendication 1 et $n_2$ est égal à 1 ou 2 selon la quantité de composé A ajoutée.

9. Procédé selon la revendication 8, caractérisé en ce que la réaction de condensation est réalisée au reflux, dans un solvant organique et en présence d'une base.

10. Procédé de préparation des composés de formule (I) où $R_1$ a la même signification que dans la revendication 1, à l'exception des valeurs suivantes: H, phényle et phényle substitué par un groupe méthoxy, trifluorométhyle ou acétyle, caractérisé en ce qu'il consiste dans la condensation du N-cyclopropylméthyl pipérazine et d'une pipérazine de formule (Ia) définie à la revendication 5, respectivement avec un composé de formule:

$$R'_1—X \qquad III$$

dans laquelle X représente un atome d'halogène et $R'_1$ a la même signification que $R_1$ dans la revendication 1, à l'exception des valeurs suivantes: H, phényle et phényle substitué par un groupé méthoxy, trifluorométhyle ou acétyle, ce qui conduit aux composés de formule:

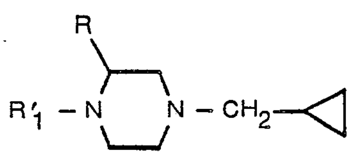 , $(A)_{n_1}$ [Ie]

où $R'_1$ et R ont la même signification que dans la formule (III) et la revendication 1 respectivement et $n_1 = 0$, ces composés de formule (Ie) étant ensuite éventuellement salifiés par addition d'un composé de formule A définie à la revendication 1 pour conduire aux composés de formule:

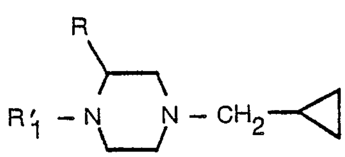 , $(A)_{n_3}$ [If]

où $R'_1$ et R ont la même signification que dans la formule (Ie), A a la même signification que dans la revendication 1 et $n_3$ est égal à 1, 2 ou 3 selon la quantité de composé A ajoutée et/ou de la réactivité de la base de formule (Ie).

11. Procédé selon la revendication 10, caractérisé en ce que lorsque dans la formule (III) $R'_1$ contient un groupe carbonyle directement relié à X, la réaction de condensation est réalisée dans un solvant organique et entre 0°C et la température ambiante.

12. Procédé selon la revendication 11, caractérisé en ce que le solvant organique est l'acétate d'éthyle.

13. Procédé selon la revendication 10, caractérisé en ce que, lorsque dans la formule (III) $R'_1$ contient un groupe $—CH_2—$ relié directement à X, la réaction de condensation est effectuée au reflux dans un solvant organique.

14. Procédé selon la revendication 13, caractérisé en ce que le solvant organique est l'isopropanol.

15. Procédé de préparation des composés de formule (I) où $R_1$ a la même signification que dans la revendication 1, à l'exception des valeurs

H et $CH_2$ 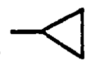

caractérisé en ce qu'il consiste dans la condensation du bromométhylcyclopropane sur une pipérazine substituée de formule:

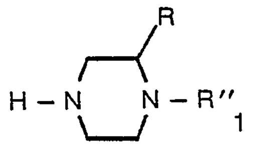

[IV]

dans laquelle R a la même signification que dans la revendication 1 et $R''_1$ a la même signification que $R_1$ dans la revendication 1, à l'exception des valeurs

— H et — $CH_2$ 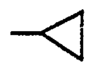

les composés résultants de formule:

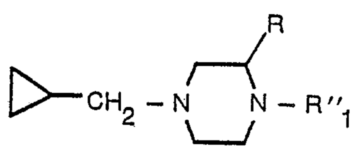 , $(A)_{n_1}$

[Ig]

où R et $R''_1$ ont la même signification que dans la formule (IV) et $n_1 = 0$, étant éventuellement salifiés par addition d'un composé de formule (A) définie dans la revendication 1, ce qui conduit aux composés de formule:

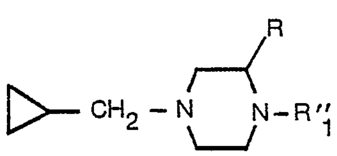 , $(A)_{n_3}$

[Ih]

dans laquelle R et A ont la même signification que dans la revendication 1, $R''_1$ a la même signification que dans la formule (IV) et $n_3$ est égal à 1, 2 ou 3 selon la quantité de composé A ajoutée.

16. Procédé selon la revendication 15, caractérise en ce que la réaction de condensation est réalisée dans un solvant organique, inerte dans les conditions de la réaction et apte à dissoudre les réactifs au reflux et en présence d'une base.

17. Procédé selon la revendication 9 ou 16, caractérisé en ce que le solvant organique est le N,N-diméthylformamide et la base est le carbonate dipotassique ou la triéthylamine.

**Patentansprüche**

1. Cyclopropylmethylpiperazine, dadurch gekennzeichnet, daß sie der allgemeinen Formel

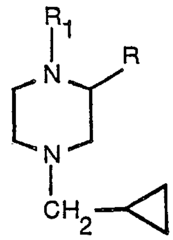 , $(A)_n$ [I]

entsprechen, worin

—R ein Wasserstoffatom oder einen Alkylrest mit 1 bis 4 Kohlenstoffatomen bedeutet,

—$R_1$

ein Wasserstoffatom,

einen geraden oder verzweigten Alkylrest mit 1 bis 5 Kohlenstoffatomen,

einen Cycloalkylmethylrest, worin die Cycloalkylgruppe 3 bis 6 Kohlenstoffatome umfaßt,

eine 2-Hydroxyethyl-Gruppe,

eine Alkoxycarbonyl-Gruppe, worin die Alkoxygruppe 1 oder 2 Kohlenstoffatome aufweist,

eine 2-Carboxylpropyl- oder 2-N-Benzylcarbamoylpropyl-Gruppe,

einen gegebenenfalls durch eine Methoxy-, Trifluormethyl- oder Acetylgruppe substituierten Phenylkern,

eine gegebenenfalls durch ein Halogenatom und/oder durch eine Methylendioxygruppe substituierte Benzyl-Gruppe,

eine gegebenenfalls durch eine Methylendioxy-Gruppe, durch 3 Methoxy-Gruppen, durch 2 Methoxy-Gruppen in 3- bzw. 5-Stellung und eine Acetoxy-Gruppe in 4-Stellung oder durch zwei Methoxy-Gruppen in 3- bzw. 5-Stellung und eine Ethoxycarbonyloxy-Gruppe in 4-Stellung substituierte Benzoyl-Gruppe,

eine Cinnamoyl-Gruppe, deren Phenylkern gegebenenfalls durch eine Trifluormethyl-Gruppe oder durch eine oder mehrere Methoxy-Gruppe(n) substituiert ist,

eine Phenoxyacetyl-Gruppe, deren Phenylkern gegebenenfalls durch ein Halogenatom oder eine Methoxy-Gruppe substituiert ist,

einen (3-Benzothienyl)-methylkern oder

einen Rest der Formel

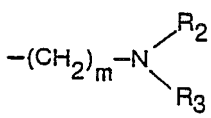 .

worin m = 2 oder 3 und $R_2$ und $R_3$ identisch sind und dann eine Alkylgruppe mit 1 bis 3 Kohlenstoffatomen bedeuten, oder zusammen mit dem Stickstoffatom, an dem sie hängen, einen Rest, ausgewählt unter Pyrrolidino, Piperidino, Hexamethylenimino, Morpholino, bilden,

bedeutet,

—A eine physiologisch annehmbare Verbindung, ausgewählt unter den Mineralsäuren, organischen Säuren, Alkylhalogeniden und Arylhalogeniden, bedeutet und

—n = 0, 1, 2 oder 3,

wobei $R_1$ jedenfalls kein Wasserstoffatom oder keinen Phenylkern bedeuten kann, wenn R = H.

2. Cyclopropylmethylpiperazine nach Anspruch 1, dadurch gekennzeichnet, daß A ausgewählt ist unter der Gruppe, umfassend Chlorwasserstoffsäure, Bromwasserstoffsäure, Schwefelsäure, Phosphorsäure, Milchsäure, Weinsäure, Brenztraubensäure, Glykolsäure, Oxalsäure, Zitronensäure, Malonsäure, Apfelsäure, Bernsteinsäure, Maleinsäure, Fumarsäure, Embonsäure, Methansulfonsäure, Methylbromid, Ethylbromid, Butylbromid, Benzylbromid.

3. Arzneimittel, insbesondere zur Behandlung von Herzschwächen und auf geringem Herzdurchsatz beruhenden Störungen, dadurch gekennzeichnet, daß es aus wenigstens einer Verbindung gemäß Anspruch 1 oder 2 besteht.

4. Medikamentöse Zusammensetzung, dadurch gekennzeichnet, daß sie wenigstens eine Verbindung gemäß Anspruch 1 oder 2 in Verbindung mit einem pharmazeutisch annehmbaren Träger enthält.

5. Verfahren zur Herstellung von Verbindungen der Formel (I), worin $R_1$ ein Wasserstoffatom bedeutet, dadurch gekennzeichnet, daß es in der Kondensation von nicht mehr als einem Mol Brommethylcyclopropan mit einem Mol piperazin der Formel

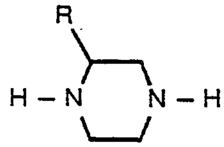

[II]

worin R einen Alkylrest mit 1 bis 4 Kohlenstoffatomen bedeutet, besteht, wobei die anfallenden Verbindungen der Formel

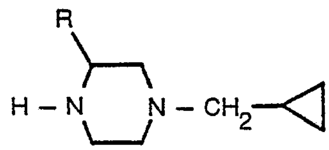 , $(A)_{n_1}$

[Ia]

worin R die gleich Bedeutung wie in der Formel (II) hat und $n_1 = 0$, dann gegebenenfalls durch Zugabe einer Verbindung der in Anspruch 1 definierten Formel A in Salze überführt werden, was zu den Verbindungen der Formel

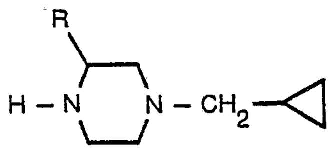 , $(A)_{n_2}$

[Ib]

führt, worin R die gleiche Bedeutung wie in der Formel (II) hat, A die gleiche Bedeutung wie in Anspruch 1 hat und $n_2$ 1 oder 2 ist, je nach der zugefügten Menge der Verbindung A.

6. Verfahren nach Anspruch 5, dadurch gekennzeichnet, daß die Kondensationsreaktion bei Raumtemperatur in einem organischen, unter den Reaktionsbedingungen inerten Lösungsmittel, das die Reaktionskomponenten zu lösen vermag, und in Gegenwart einer Base durchgeführt wird.

7. Verfahren nach Anspruch 6, dadurch gekennzeichnet, daß das organische Lösungsmittel Ethylacetat und die Base Kaliumcarbonat oder Tri-ethylamin ist.

8. Verfahren zur Herstellung von Verbindungen der Formel (I), worin $R_1$ eine Cyclopropylmethyl-Gruppe bedeutet, dadurch gekennzeichnet, daß es in der Kondensation von wenigstens zwei Mol Brommethylcyclopropan mit einem Mol Piperazin bzw. einem Mol der Verbindung der Formel (II) besteht, wobei die anfallenden Verbindungen der Formel

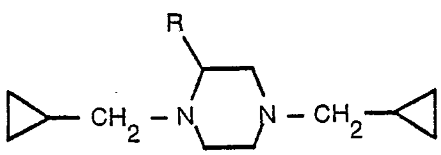 , $(A)_{n_1}$

[Ic]

worin R die gleiche Bedeutung wie im Anspruch 1 hat und $n_1 = 0$, dann gegebenenfalls durch Zugabe einer Verbindung der im Anspruch 1 definierten Formel A in Salze übergeführt werden, was zu den Verbindung der Formel

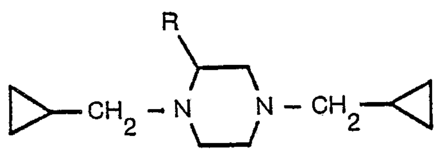 , $(A)_{n_2}$

[Id]

führt, worin R und A die gleiche Bedeutung wie in Anspruch 1 haben und $n_2$ 1 oder 2 ist, je nach der zugesetzten Menge der Verbindung A.

9. Verfahren nach Anspruch 8, dadurch gekennzeichnet, daß die Kondensationsreaktion bei Rückfluß in einem organischen Lösungsmittel und in Gegenwart einer Base durchgeführt wird.

10. Verfahren zur Herstellung von Verbindungen der Formel (I), worin $R_1$ die gleiche Bedeutung wie im Anspruch 1 hat, mit Ausnahme der folgenden Bedeutungen: H, Phenyl und durch eine Methoxy-, Trifluormethyl- oder Acetyl-Gruppe substituiertes Phenyl, dadurch gekennzeichnet, daß es in der Kondensations des N-Cyclopropylmethylpiperazins bzw. eines Piperazins der in Anspruch 5 definierten Formel (Ia) mit einer Verbindung der Formel

$$R'_1—X \qquad (III),$$

worin X ein Halogenatom bedeutet und $R'_1$ die gleiche Bedeutung wie $R_1$ in Anspruch 1 hat, mit Ausnahme der folgenden Bedeutungen: H, Phenyl und durch eine Methoxy-, Trifluormethyl- oder Acetyl-Gruppe substituiertes Phenyl, besteht, was zu den Verbindungen der Formel

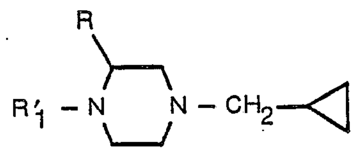 , $(A)_{n_1}$ [Ie]

führt, worin $R'_1$ und R die gleiche Bedeutung wie in der Formel (III) bzw. in Anspruch 1 haben und $n_1 = 0$, wobei diese Verbindungen der Formel (Ie) sodann gegebenenfalls durch Zugabe einer Verbindung der in Anspruch 1 definierten Formel A in Salze übergeführt werden, um zu Verbindungen der Formel

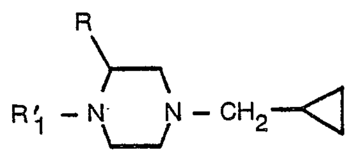 , $(A)_{n_3}$ [If]

zu führen, worin $R'_1$ und R die gleiche Bedeutung wie in der Formel (Ie) haben, A die gleiche Bedeutung wie in Anspruch 1 hat und $n_3$ 1, 2 oder 3, je nach der zugestezten Menge der Verbindung A und/oder der Reaktivität der Base der Formel (Ie), ist.

11. Verfahren nach Anspruch 10, dadurch gekennzeichnet, daß, wenn in der Formel (III) $R'_1$ eine direkt an X hängende Carbonylgruppe enthält, die Kondensationsreaktion in einem organischen Lösungsmittel und zwischen 0°C und Raumtemperatur durchgeführt wird.

12. Verfahren nach Anspruch 11, dadurch gekennzeichnet, daß das organische Lösungsmittel Ethylacetat ist.

13. Verfahren nach Anspruch 10, dadurch gekennzeichnet, daß, wenn in der Formel (III) $R'_1$ eine direkt an X hängende —$CH_2$-Gruppe enthält, die Kondensationsreaktion bei Rückfluß in einem organischen Lösungsmittel durchgeführt wird.

14. Verfahren nach Anspruch 13, dadurch gekennzeichnet, daß das organische Lösungsmittel Isopropanol ist.

15. Verfahren zur Herstellung von Verbindungen der Formel (I), worin $R_1$ die gleiche Bedeutung wie im Anspruch 1 hat, mit Ausnahme der Bedeutungen

H und $CH_2$ 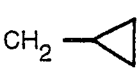

dadurch gekennzeichnet, daß es in der Kondensation von Brommethylcyclopropan mit einem substituierten Piperazin der Formel

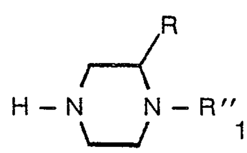 [IV]

worin R die gleiche Bedeutung win im Anspruch 1 hat und $R''_1$ die gleiche Bedeutung wie $R_1$ im Anspruch 1 hat, mit Ausnahme der Bedeutungen

—H und — $CH_2$ 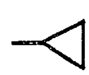

besteht, wobei die anfallenden Verbindung der Formel

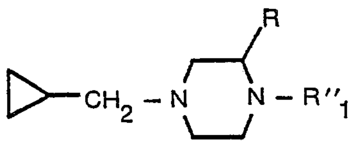 , $(A)_{n_1}$ [Ig]

worin R und $R''_1$ die gleich Bedeutung wie in der Formel (IV) haben und $n_1 = 0$, gegebenenfalls durch Zugabe einer Verbindung der in Anspruch 1 definierten Formel (A) in Salze übergeführt werden, was zu den Verbindungen der Formel

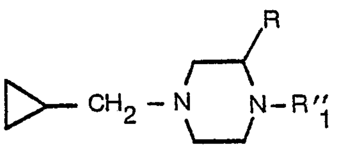 , $(A)_{n_3}$ [Ih]

führt, worin R und A die gleiche Bedeutung wie im Anspruch 1 haben, $R''_1$ die gleiche Bedeutung wie in der Formel (IV) hat und $n_3$ 1, 2 oder 3, je nach der zugesetzten Menge der Verbindung A, ist.

16. Verfahren nach Anspruch 15, dadurch gekennzeichnet, daß die Kondensationsreaktion in einem organischen Lösungsmittel, das unter den Reaktionsbedingungen inert ist und die Reaktionskomponenten zu lösen vermag, bei Rückfluß und in Gegenwart einer Base durchgeführt wird.

17. Verfahren nach Anspruch 9 oder 16, dadurch gekennzeichnet, daß das organische Lösungsmittel N,N-Dimethylformamid und die Base Kaliumcarbonat oder Triethylamin ist.

**Claims**

1. Cyclopropylmethylpiperazines, characterized in that they are represented by general formula:

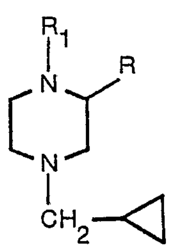 , $(A)_n$ [I]

in which:

—R represents a hydrogen atom or an alkyl radical having 1 to 4 carbon atoms;

—$R_1$ represents:

a hydrogen atom,

a straight or branched alkyl radical having 1 to 5 carbon atoms

a cycloalkylmethyl radical wherein the cycloalkyl group comprises 3 to 6 carbon atoms,

a 2-hydroxyethyl group,

an alkoxycarbonyl group wherein the alkoxy group has 1 or 2 carbon atoms,

a 2-carboxypropyl or N-benzyl 2-carbamoylpropyl group,

a phenyl ring which may be substituted by a methoxy, trifluoromethyl or acetyl group,

a benzyl group which may be substituted by a halogen atom and/or by a methylenedioxy group,

a benzoyl group which may be substituted by a halogen atom and/ or by a methylenedioxy group,

a benzoyl group which may be substituted by a methylenedioxy group; by three methoxy groups; by two methoxy groups respectively in 3- and 5- positions and an acetoxy group in 4- position; or by two methoxy groups respectively in 3- and 5- positions and an ethoxycarbonyloxy group in 4- position,

a cinnamoyl group, the phenyl ring of which may be substituted by a trifluoromethyl group or by one or more methoxy groups,

a phenoxyacetyl group, the phenyl ring of which may be substituted by a halogen atom or a methoxy group,

a (3-benzothienyl methyl ring, or

a radical of formula

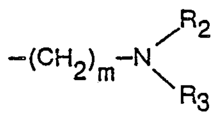

in which m = 2 or 3 and $R_2$ and $R_3$ are identical and represent then an alkyl group of 1 to 3 carbon atoms or form together with the nitrogen atom to which they are linked, a radical selected from the following: pyrrolidino, piperidino, hexamethyleneimino, morpholino;

—A represents a physiologically acceptable compound selected from inorganic acids, organic acids, alkyl halides and aryl halides; and

—n = 0, 1, 2 or 3;

with the proviso that $R_1$ cannot represent a hydrogen atom or a phenyl ring when R = H.

2. Cyclopropylmethyl piperazines according to claim 1, characterized in that A is selected from the group comprising hydrochloric acid, hydrobromic acid, sulfuric acid, phosphoric acid, lactic acid, tartric acid, pyruvic acid, glycolic acid, oxalic acid, citric acid, malonic acid, malic acid, succinic acid, maleic acid, furmaric acid, embonic acid, methanesulfonic acid, methyl bromide, ethyl bromide, butyl bromide, benzyl bromide.

3. Drug particularly for the treatment of cardiac failures and troubles due to a low cardiac flow, characterized in that it consists of at least one compound according to claim 1 or 2.

4. Medicinal composition, characterized in that it contains at least one compound according to claim 1 or 2, in combination with a pharmaceutically acceptable carrier.

5. Process for preparing compounds of formula (I) in which $R_1$ represents a hydrogen atom, characterized in that it comprises condensing not more than 1 mole of bromomethylcyclopropane on 1 mole of piperazine of formula:

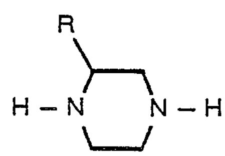 [II]

in which R is an alkyl radical of 1 to 4 carbon atoms, the resulting compounds of formula:

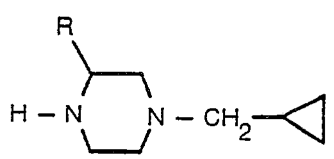 , $(A)_{n_1}$ [Ia]

in which R has the same meaning as in formula (II) and $n_1 = 0$, being then possibly salified by addition of a compound of formula (A) defined in claim 1, thus obtaining compounds of formula:

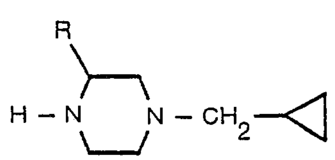 , $(A)_{n_2}$ [Ib]

in which R has the same meaning as in formula (II), A has the same meaning as in claim 1, and $n_2$ is equal to 1 or 2 according to the amount of added compound A.

6. Process according to claim 5, characterized in that the condensation reaction is carried out at room temperature, in an organic solvent inert in the conditions of the reaction and capable of solubilizing the reactants and in the presence of a base.

7. Process according to claim 6, characterized in that the organic solvent is ethyl acetate and the base is dipotassium carbonate or triethylamine.

8. Process for preparing compounds of formula (I) in which $R_1$ represents a cyclopropylmethyl group, characterized in that it comprises condensing at least 2 moles of bromomethylcyclopropane respectively on 1 mole of piperazine and 1 mole of compound of formula (II), the resulting compounds of formula:

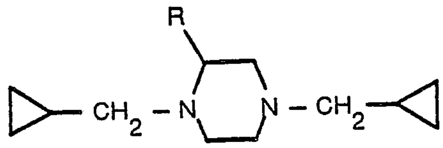 , $(A)_{n_1}$ [Ic]

in which R has the same meaning as in claim 1 and $n_1 = 0$, being then possibly salified by addition of a compound of formula A defined in claim 1, thus obtaining compounds of formula:

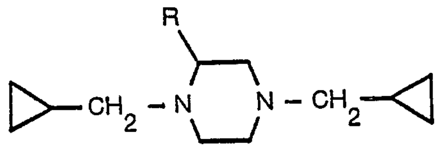 , $(A)_{n_2}$ [Id]

in which R and A have the same meanings as in claim 1 and $n_2$ is equal to 1 or 2 according to the amount of added compound A.

9. Process according to claim 8, characterized in that the condensation reaction is carried out at reflux in an organic solvent and in the presence of a base.

10. Process for preparing compounds of formula (I) in which $R_1$ has the same meaning as in claim 1, with the exception of the following values: H, phenyl and phenyl substituted by a methoxy trifluoromethyl or acetyl group, characterized in that it comprises condensing N-cyclopropylmethyl piperazine and a piperazine of formula (Ia) defined in claim 5, respectively with a compound of formula:

$$R'_1—X \qquad (III)$$

in which X represents a halogen atom and $R'_1$ has the same meaning as $R_1$ in claim 1, with the exception of the following values: H, phenyl and phenyl substituted by a methoxy, trifluoromethyl or acetyl group, thus giving compounds of formula:

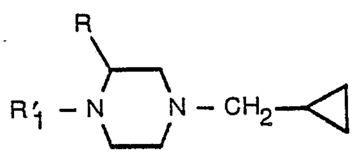 , $(A)_{n_1}$ [Ie]

in which $R'_1$ and R have the same meanings as in formula (III) and claim 1 respectively and $n_1 = 0$, these compounds of formula (Ie) being then possibly salified by addition of a compound of formula A defined in claim 1 for obtaining compounds of formula:

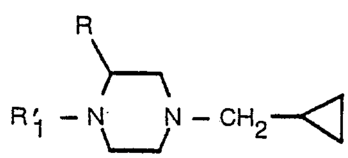 , $(A)_{n_3}$ [If]

in which $R'_1$ and R have the same meanings as in formula (Ie), A has the same meaning as in claim 1 and $n_3$ is equal to 1, 2 or 3 according to the added amount of compound A and/or the reactivity of the base of formula (Ie).

11. Process according to claim 10, characterized in that when in formula (III) $R'_1$ contains a carbonyl group directly linked to X, the condensation reaction is carried out in an organic solvent and between 0°C and the room temperature.

12. Process according to claim 11, characterized in that the organic solvent is ethyl acetate.

13. Process according to claim 10, characterized in that when in formula (III) $R'_1$ contains a $—CH_2—$ group directly linked to X, the condensation reaction is carried out at reflux in an organic solvent.

14. Process according to claim 13, characterized in that the organic solvent is isopropanol.

15. Process for preparing compounds of formula (I) in which $R_1$ has the same meaning as in claim 1, with the exception of the values

H and $CH_2$ 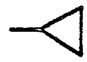

characterized in that it comprises condensing bromomethylcyclopropane on a substituted piperazine of formula:

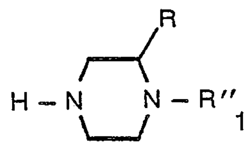 [IV]

in which R has the same meaning as in claim 1 and $R''_1$ has the same meaning as $R_1$ in claim 1 with the exception of the values

— H and — $CH_2$ 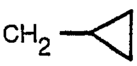

the resulting compounds of formula:

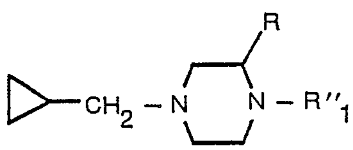 , $(A)_{n_1}$ [Ig]

in which R and $R''_1$ have the same meanings as in formula (IV) and $n_1 = 0$, being possibly salified by addition of a compound of formula (A) defined in claim 1, thus obtaining compounds of formula:

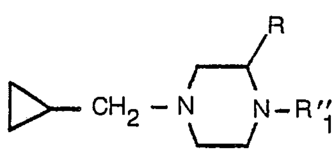 , $(A)_{n_3}$ [Ih]

in which R and A have the same meanings as in claim 1, $R''_1$ has the same meaning as in formula (IV) and $n_3$ is equal to 1, 2 or 3 according to the added amount of compound A.

16. Process according to claim 15, characterized in that the condensation reaction is carried out in an organic solvent inert in the conditions of the reaction and capable of dissolving the reactants at reflux and in the presence of a base.

17. Process according to claim 9 or 16, characterized in that the organic solvent is N,N-dimethylformamide and the base is dipotassium carbonate or triethylamine.